# EUROPÄISCHE PATENTSCHRIFT

(11) **EP 2 928 286 B1**
(45) Veröffentlichungstag und Bekanntmachung des Hinweises auf die Patenterteilung: **03.01.2018**
(21) Anmeldenummer: 13811782.5
(22) Anmeldetag: 27.11.2013
(51) Int. Cl.: A01G 33/00, A01G 9/18, C02F 3/30

(54) **VERFAHREN ZUM BETRIEB EINER AQUATISCHEN PFLANZENKULTUREINRICHTUNG FÜR FUTTERMITTEL, SOWIE PFLANZENKULTUREINRICHTUNG SELBST**
METHOD FOR OPERATING AN AQUATIC PLANT CULTURE UNIT FOR FEEDING STUFFS, AND PLANT CULTURE UNIT ITSELF
PROCÉDÉ POUR FAIRE FONCTIONNER UN DISPOSITIF DE CULTURE DE PLANTES AQUATIQUES POUR DES FOURRAGES, AINSI QUE DISPOSITIF DE CULTURE DE PLANTES AQUATIQUES LUI-MÊME

(30) Priorität: 06.12.2012 DE 102012023929
(43) Veröffentlichungstag der Anmeldung: 14.10.2015
(73) Patentinhaber: Rogmans, Maria, 47546 Kalkar (DE)
(72) Erfinder: WILHELM, Hermann-Josef, 47546 Kalkar (DE)
(74) Vertreter: Schmidt, Karl Michael
(86) Internationale Anmeldenummer: PCT/EP2013/003577
(87) Internationale Veröffentlichungsnummer: WO 2014/086465

(56) Entgegenhaltungen:
- WO-A2-2010/043323
- WO-A2-2011/047778
- DE-A1-102011 012 676
- CHENG JAY J ET AL: "Growing Duckweed to Recover Nutrients from Wastewaters and for Production of Fuel Ethanol and Animal Feed", CLEAN - SOIL, AIR, WATER, WILEY - VCH VERLAG GMBH & CO. KGAA, DE, Bd. 37, Nr. 1, 26. Januar 2009 (2009-01-26), Seiten 17-26, XP002621890, ISSN: 1863-0650, DOI: 10.1002/CLEN.200800210 [gefunden am 2009-01-22]

## Beschreibung

Die Erfindung betrifft ein Verfahren zum Betrieb einer aquatischen Pflanzenkultureinrichtung für Futtermittel, sowie Pflanzenkultureinrichtung selbst, gemäß Oberbegriff der Patentansprüche 1 und 8.
Eine Kultureinrichtung für aquatische Pflanzen ist aus der WO 2010/043323 bekannt, bei der insbesondere bei der Verwendung von Schwachlichtpflanzen eine Anordnung von in Regalen übereinander, d.h. in Etagen angeordneten Kulturwasserwannen vorgesehen ist, in der die aquatischen Pflanzen kultiviert und aus denselben geerntet werden. Aus der DE 10 2011 012 676 A1 ist eine Kultureinrichtung bekannt, bei welcher das Kulturwasser mit konditioniertem Ausgangswasser gespeist und so reproduzierbar gedüngt wird.
Aus Kultureinrichtungen dieser Art erfolgt eine zyklische Beerntung, wie dies bei solchen Kultureinrichtungen aus der WO 2011/047778 A2 bekannt ist.

Aquatische Pflanzen wie Wasserlinsen, Wasserhyazinthen etc sind in erheblichem Maße als Futtermittel oder als proteinreicher Futtermittelzusatz für Tierfutter geeignet.
Übliche Futterpflanzen wie Mais und Soja sind Landpflanzen und stehen daher nur in den Erntezeiten zur Verfügung. Das heisst, bei einer oder zwei Ernten im Jahr steht diese Futterpflanze zur Verfügung und muss sodann verarbeitet werden.
D.h. innerhalb kürzester Zeit müssen große Erntemengen verarbeitet werden, oder man nimmt in Kauf, dass Futterpflanzen global aus verschiedenen Jahreszeitenzonen über weite Strecken transportiert werden müssen.
Der Erfindung liegt daher die Aufgabe zugrunde, dass einerseits Futter, oder Futtermittelpflanzen vor Ort möglichst ganzjährig frisch zur Verfügung stehen sollen, und dass die Beerntung und Futtermittelproduktion erheblich besser aufeinander abgestimmt sind, und dabei zugleich die Resourcen Landbedarf, Wasserbedarf und Energiebedarf bei der Pflanzenproduktion minimiert wird. Die gestellte Aufgabe wird bei einem Verfahren der gattungsgemäßen Art durch die kennzeichnenden Merkmale des Anspruches 1 gelöst.
Weitere vorteilhafte Ausgestaltungen sind in den abhängigen Ansprüchen 2 bis 7 angegeben. Im Hinblick auf einer Einrichtung mit aquatische Kultureinrichtung ist die gestellte Aufgabe erfindungsgemäß durch die kennzeichnenden Merkmale des Anspruches 8 gelöst. Weitere vorteilhafte Ausgestaltungen sind in den übrigen abhängigen Ansprüchen angegeben.
Kern der verfahrensgemäßen Erfindung ist, dass der Pflanzenproduktion im gestapelten Aquakultursystem eine damit zeitlich korrelierte und koordinierte Futtermittelproduktion und/oder Futtermittelkonditionierung beigeordnet ist, derart, dass die Beerntungsmengen der Aquakulturen an die nachfolgende Futtermittelproduktion automatisch in der Weise angepasst ist, dass die in Summe täglich oder wöchentlich beernteten Mengen aquatischer Pflanzen in situ zumindest nach teilweiser Auftrocknung der Pflanzen zu Futtermittel verarbeitet werden können.
Ein solches Aquakultursystem ist geschlossen, also in einem gewächshausähnlichen Photobioreaktor angeordnet. Dabei sind durch die Verwendung aquatischer Pflanzen von Jahreszeitenunabhängige Kultur- und Erntezeiten gegeben. Ferner sind aquatische Pflanzen extrem schnellwüchsig und selbstvermehrend.
Die Kulturflächen können daher mit täglichen Partialmengen von 8% bis 50% der maximalen Bedeckung beerntet werden. D.h die Biomasse steht ganzjährig FRISCH zur Verfügung. Der oben genannte Mengenbereich in der Beerntung ist relativ weit.
Dabei nutzt die vorliegende Erfindung den Effekt aus, dass die Nachwuchsstimulation der Kulturen, bspw von Lemnacea und Eichhornia genau von der entnommenen Erntemenge wesentlich mit beeinflusst wird.

Dies nutzt die Erfindung durch die direkte und automatische Anpassbarkeit des nachfolgenden Futtermittel-Produktionsprozesses an die täglichen Erntemenge und umgekehrt.
Damit wird das verwendete aquatische Kultursystem zu einem "lebenden bedarfsgeregelten Frischlager" mit allzeit frischer Biomasse.
Insgesamt entstehten folgende kumulativen Vorteile:
Ressourcenschonung und Nachhaltigkeit weil:
   - Kompakte Pflanzenproduktion ohne Bedarf landwirtschaftlicher Flächen;
   - Jahreszeitenunabhängige ganzjährige, vor allem ganzjährig vor Ort verfügbare Frischmasse ohne Transportwege;
   - Erheblich geringerer Wasserbedarf;
   - Geringer Energieeinsatz und damit CO2-minimiert.
In vorteilhafter Ausgestaltung ist angegeben, dass die aquatische Pflanzenkultureinrichtung in Zonen unterschiedlicher Temperatur und/oder unterschiedlicher Wärme im Kulturwasser, und/oder unterschiedlicher Ausleuchtung oder Kunstlicht unterschiedlicher Wellenllänge und/oder unterschiedlicher Kulturwasserzusammensetzung unterteilt ist, in welchen in den aquatischen Pflanzen durch Steuerung dieser Parameter unterschiedliche Inhaltsstoffspektren generierbar sind. Dies betrifft im Wesentlichen aber nicht ausschließlich die Verwendung von Wasserlinsen unterschiedlichster Sorten. Diese Wasserlinsen reagieren auf die oben genannten Variationen der oben genannten Parameter mit entsprechender Verschiebung des Inhaltsstoffspektrums. Dabei geht es im Wesentlichen um die Protein- und Stärkegehalte, die in diesen Pflanzen mit 35% bis über 40% sehr hoch liegen. Eine entsprechende Austarierung von Stärke gegenüber Protein, kann so je nach Anforderung für das gewünschte final hergestellte Futtermittel eingestellt werden. Insbesondere sind die Inhaltsstoffspektren bei entsprechender Steuerung reproduzierbar. So kann die Biomasse exakt auf die gewünschte finale Futtermittelauslegeung auf Proteine etc designed/gestaltet werden.

Die hierzu in der Kultureinrichtung geschaffenen Zonen können als abgeteilte Abteilungen betrieben werden.

Wichtiger Faktor ist ebenso, dass die Kultureinrichtung mit Wasserlinsen vorzugsweise in sortenreinen Kulturen, und nicht in Mischkulturen betrieben werden. Dies ist von ganz erheblicher Bedeutung für den erfindungsgemäßen Betrieb der Einrichtung.
Das gewährleistet den stabilsten Ertrag und die beste Reproduzierbarkeit im Hinblick auf die gewünschten eingestellten Inhaltsstoffe.
Außerdem ist dies vorteilhaft in Bezug auf die wertvolle Biozertifizierung.

Mit dieser Ausgestaltung kann dies sogar zonenweise erfolgen, so dass innerhalb einer aquatischen Kultureinrichtung jeweils gewünschte verschiedene Biomasse direkt appliziert auf die nachfolgende Futtermittelproduktion eingestellt werden kann.

Weiterhin ist daher vorteilhaft ausgestaltet, dass zonenweise geerntet wird und die dort jeweils reproduzierbar unterschiedlichen Inhaltsstoffe nachfolgend entsprechend in die Futtermittelproduktion eingehen, um jeweils anforderungsnah designte Biomasse als Rohstoffe zu generieren.

Bei den eingesetzten Wasserlinsen handelt es sich um konventionell gezüchtete Sorten, die keiner gentechnischen Behandlung unterworfen wurden und somit GMO-frei sind.

Mit Hilfe der geschlossenen oben genannten aquatischen Kultureinrichtungen ist dies möglich, so dass diese Freiheit von Gentechnik erheblich vorteilhaft für das daraus resultierte Futtermittel ist.

In weiterer vorteilhafter Ausgestaltung ist angegeben, dass die bei der Kultivierung benötigten Wassermengen zumindest teilweise aus der Kondensation von Kulturwasserverdunstungsmengen im Photobioreaktor und/oder aus der Auftrocknung der entnommenen Pflanzen wieder in die Kultureinrichtung des Photobioreaktors zurückgeführt werden. Auf diese Weise wird diese Biomasse mit in einem Vollzyklus gefahrenen Wassers kultiviert. Im Prinzip muss nur am Anfang eine Startmenge an Kulturwasser aus Brunnenwasser etc bereitsgestellt werden. Sodann fährt das Wasser im Prozess, bis hin zur Trocknung, immer im Kreis und es müssen nur Wasserverluste nachgefüllt werden. Damit ist dieser Biomasse-Produktionsprozess wegen des vorgegebenen geschlossenen Kultursystems konsequenterweise auch extrem nachhaltig da wassersparend.

Dadurch, dass nunmehr die Produktion von Frischmasse und die Futtermittelproduktion aus der Frischmasse auch räumlich zusammengeführt sind, kann auch im Übrigen extrem ressourcenschonend ein zudem grundsätzlich hochwertiges Futtermittel produziert werden.

In weiterer vorteilhafter Ausgestaltung ist vorgesehen, dass die aquatische Kultureinrichtung mittels Abwärme aus dem Futtermittelproduktionsprozess oder mittels Solarthermie beheizt wird. Auf diese Weise entsteht eine weitere Komponente der Nachhaltigkeit dieser spezifischen Futtermittelproduktion.

In weiterer vorteilhafter Ausgestaltung ist angegeben, dass am Frischmasseausschleusungspunkt der aquatischen Kultureinrichtung ein geschlossenes lichtdurchlässig eingehaustes Areal zur solarthermischen Vortrockung der Frischmasse eingesetzt wird.

In weiterer vorteilhafter Ausgestaltung ist angegeben, dass das Kondenswasser aus der aquatischen Kultureinrichtung oder der Vortrocknung in das Kulturwasser der aquatischen Kultureinrichtung rückgeführt führt, indem es kurz vor Zuleitung in die Kulturflächen mit einer Entkeimung behandelt wird. Auf diese Weise lässt sich der Prozess mit hoher Hygiene und dauerhafter Qualität fahren.

In weiterer vorteilhafter Ausgestaltung ist angegeben, dass landwirtschaftliche Gülle dem Kulturwasser beigemischt wird, bevor es entkeimt und nachfolgend der Kultureinrichtung zugeleitet wird. D.h. dass die fertige Gülle/Kulturwassermischung entkeimt wird. Nachfolgend wird dies dann den Kulturen zugeführt.

In weiterer vorteilhafter Ausgestaltung ist angegeben, dass zuvor aus landwirtschaftlicher Gülle oder Gärsubstraten die Dickphase (feste sedimentierbare Bestandteile) von der Gülle getrennt wird, und die so erhaltene Dünnphase dem Kulturwasser beigemischt wird. Auf diese Weise wird eine Sedimentation in der Pflanzenkulturanlage vermieden wird. Die abgetrennte Dickphase kann verschiedensten Anwendungen zugeführt werden.
In Bezug auf die erfindungsgemäße Einrichtung besteht der Kern der Erfindung darin, dass eine geschlossene aquatische Pflanzenkultureinrichtung mit übereinander gestapelten Kulturwannen eines Aquakultursystems vorgesehen ist, die einer Futtermittelproduktionseinrichtung räumlich beigeordnet oder zugeordnet ist, derart, dass eine damit zeitlich korrelierte und koordinierte Futtermittelproduktion erfolgt, bei welcher die Beerntungsmengen der Aquakulturen auf die nachfolgende Futtermittelproduktion automatisch steuerbar ist et vice versa, und die so in Summe täglich oder wöchentlich beernteten Mengen aquatischer Pflanzen *in situ* zumindest nach teilweiser Auftrocknung der Pflanzen direkt zu Futtermittel verarbeitbar sind.
Eine räumliche Beiordnung beider Anlagen, nämlich der aquatischen Pflanzenkultureinrichtung und der Futtermittelproduktionseinrichtung ist daher vorteilhaft. Dies ist aber nur eine mögliche Variante des Betriebes. Pflanzenkultureinrichtung und Futtermittelproduktionseinrichtung brauchen aber auch nicht direkt räumlich beigeordnet zu sein. Es genügt, wenn eine "anderswo" platzierte aquatische Pflanzenkultureinrichtung mit einer Futtermittelproduktionseinrichtung zusammen disponiert wird.

Dies ist mit der besagten Erfindung deshalb möglich, weil aus dieser besonderen aquatischen Pflanzenkultureinrichtung ein ganzjähriger Betrieb mit täglicher oder mehrmals wöchentlicher oder monatlicher regelmäßiger Beerntung möglich ist. Anders als bei Landpflanzen steht das Pflanzenmaterial somit zeitlich sowie mengenmäßig quasi unbegrenzt zur Verfügung.
Somit kann auch die der Pflanzenkultureinrichtung disponiert zugewiesene Futtermittelproduktionseinrichtung konstant ganzjährig ausgelastet werden. Das ist der neben der qualitätiv extrem hochwertigen Biomasse weitere erhebliche Vorteil der Erfindung.

In weiterer vorteilhafter Ausgestaltung ist angegeben, dass am Frischmasseausschleusungspunkt der aquatischen Pflanzenkultureinrichtung ein geschlossenes eingehaustes Areal zur solarthermischen oder prozessabwärmegespeisten Vortrockung der Frischmasse vorgesehen ist.

Ebenso sind Wärmebeiträge aus Prozessabwärme bspw aus der Futtermittelproduktion und/oder geothermale Wärme, und/oder Grundwasser oder Thermalquellenwärme insbesondere im niederkalorischen, je nach geographischem Einsatzort einsetzbar, um die Biomassenertäge in der Kultureinrichtung zu optimieren.

In weiterer vorteilhafter Ausgestaltung ist angegeben, dass die Kultureinrichtung sowie das zur Vortrockung vorgesehene Areal mit Kondensationsflächen versehen sind, die flüssigkeits- und/oder tropfschlüssig mit einem Kondensationswassersammelbehältnis verbunden sind, so dass die bei der Kultivierung benötigten Wassermengen zumindest teilweise aus der Kondensation von Kulturwasserverdunstungsmengen im Photobioreaktor und/oder aus der Auftrocknung der entnommenen Pflanzen wieder in die Kultureinrichtung des Photobioreaktors zurückgeführt wird. Auf diese Weise ist in final einfacher Weise die wasserminimierte Produktion von Futtermittel gewährleistet.

In weiterer vorteilhafter Ausgestaltung ist angegeben, dass die aquatische Kultureinrichtung mittels Abwärme aus dem Futtermittelproduktionsprozess oder mittels Solarthermie beheizt wird. Hierdurch ist eine nachhaltige Nutzung von niederkalorischer ansonsten verlorener Prozessabwärme gegeben. Die Beheizung der Pflanzenkultureinrichtung hat wiederum eine Steigerung des Biomassenertrages zur Folge.

Weiterhin ist vorteilhaft ausgestaltet, dass am Frischmasseausschleusungspunkt der aquatischen Kultureinrichtung ein geschlossenes lichtdurchlässig eingehaustes Areal zur solarthermischen Vortrocknung der Frischmasse eingesetzt wird.

Ebenso vorteilhaft, wie oben bereits im Verfahren beschrieben, ist, dass das Kondenswasser aus der aquatischen Kultureinrichtung oder der Vortrocknung in das Kulturwasser der aquatischen Kultureinrichtung rückgeführt wird, indem es kurz vor Zuleitung in die Kulturflächen mit einer Entkeimung behandelt wird.

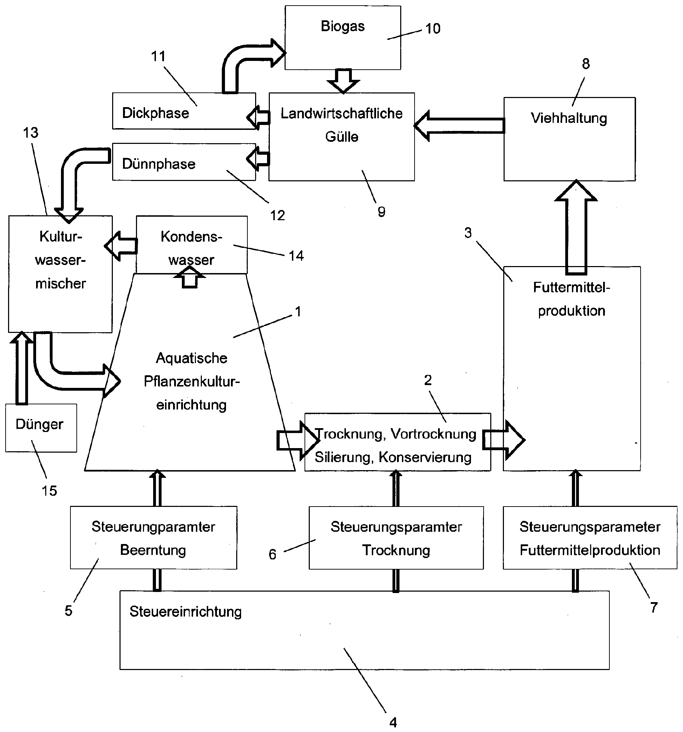

Zur Ernährung der aquatischen Pflanzenkulturen ist vorteilhaft ausgestaltet, dass landwirtschaftliche Gülle dem Kulturwasser beigemischt wird, bevor es entkeimt und nachfolgend der Kultureinrichtung zugeleitet wird. Dabei ist, wenn gefordert, ein hoher Hygienestandard möglich. Vorteilhaft ist außerdem, dass zuvor aus landwirtschaftlicher Gülle die Dickphase (feste sedimentierbare Bestandteile) von der Gülle getrennt wird, und die so erhaltene Dünnphase dem Kulturwasser über einen Mischer beigemischt wird.
Ein Ausgestaltungsbeispiel der Erfindung ist in der Zeichnung dargestellt und nachfolgend näher erläutert. Ausgehend von einer aquatischen Pflanzenkultureinrichtung 1 mit einem System von gestapelten Aquakulturwannen, wie sie bspw aus der WO 2010 /043323 A2 bekannt ist, werden dort aquatische Pflanzen, nämlich im Wesentlichen aber ggfs nicht ausschließlich Wasserlinsen kultiviert. Die Wasserlinsen eignen sich aber aufgrund ihrer Schwachlichteigenschaft ganz besonders, um diese in sich abschattenden gestapelten Wannenkulturen zu kultivieren. Dort werden die aquatischen Pflanzen täglich, oder mehrmals täglich bis wöchentlich oder mehrmals wöchentlich geerntet. Die Beerntung kann bspw über ein Floatingsystem gesteuert vorgenommen werden. Dabei können Erntezeitpunkte sowie Erntemengen pro Kulturflächen gesteuert werden. Idealerweise wird jede der Kulturflächen/Wannen im oben genannten wiederkehrenden Zeitraum beerntet. Der Grund dafür ist, dass aquatische Pflanzen bis zu einer maximalen Grenzbedeckung wachsen und dann den Wuchs stoppen, bis durch Beerntung die Populationsdichte wieder deutlich unter die Grenzbedeckung gefahren wird. Mit anderen Worten ist die Beerntung der Kulturflächen gleichzeitig wuchstimulierend. Das heisst aber, dass man über die Steuerung der Beerntungsmengen und -Zyklen gezielt Einfluss auf die anfallende verfügbare nachwachsende Biomasse hat.
Dieser mögliche Steuereingriff ist wesentlich für die Erfindung. Die geernteten Mengen an Biomasse werden nun am Auslauf der Kultureinrichtung 1 über eine thermisch oder solarthermisch betriebene Trocknung oder Vortrocknung 2 geschleust. Diese kann bei räumlich dichter Anordnung von Kultureinrichtung 1 und Futtermittelproduktion 3 auch thermisch aus Prozessabwärme gespeist sein. Beide Prozesse können über entweder eine gemeinsame Steuerung geführt werden, wenn die Kultureinrichtung 1 und die Futtermittelproduktionseinrichtung räumlich beieinander liegen.

Wenn aber Kultureinrichtung 1 und Futtermittelproduktion 3 räumlich nicht direkt beieinander liegen, dann können trotzdem aufeinander abgestimmte Steuerungsparameter für die Beerntung 5 und Steuerungsparameter für die Futtermittelproduktion generiert werden. Auch wenn diese in zwei getrennten Steuerungen vorgenommen werden. Eine koordinierte Ernte/Produktion kann aber auch via Internet koodiniert werden.

Auf diese Weise ist es möglich, dass ausgewählte Kultureinrichtungen im ganzjährigen Betrieb so gesteuert werden, dass deren jeweils zugewiesene Futtermittelproduktionsstätte genau auf diese anfallende Biomassenmenge ausgelegt und gesteuert ist.

Eine solche Koordination ist mit Landpflanzen bisher gar nicht möglich, weil der anfallende Biomassenertrag bei Landpflanzen unstetig (1 bis 3mal im Jahr) ist, und außerdem die Erntemenge bei Landpflanzen immer komplett geerntet und dann auf Halde oder in Silos zwischengelagert werden muss.

Dies ist im vorliegenden erfindungsgemäßen Fall gar nicht nötig und kann daher vollständig entfallen.
Die für die Futtermittelproduktion anfallende Biomassenmenge also die Erntemenge wird somit täglich insitu auf die Kapazität der Futtermittelproduktion direkt angepasst.

Mit einbezogen in die Steuerung kann demnach auch die Trocknung/Vortrocknung 6 sein, so dass auch diese auf den weiteren Prozess zeitlich koordiniert erfolgt, so dass Wartezeiten in der Trocknung zeitlich mit berücksichtigt werden.

Anstatt oder zusätzlich zur Trocknung oder Vortrocknung kann die Biomasse aus siliert werden oder aber auf andere Weise sogar nasskonditioniert werden, je nachdem, was als Eingangsrohstoff für die nachfolgende Futtermittelproduktion gewünscht ist. Aber auch diese optionalen Schritte erfolgen mit einer hohen Reproduzierbarkeit.

Von der Futtermittelproduktion wird das Futter sodann zur Viehhaltung 8 verbracht und dort verfüttert. Die Viehhaltung liefert sodann Gülle. Diese Gülle aus der Viehhaltung und ggfs auch Gärreste aus Biogasanlagen 10 bilden insgesamt die sogenannte landwirtschaftliche Gülle 9.

Sodann erfolgt vorteilhafterweise aber nicht zwingend eine Abtrennung von Dickphase 11 und Dünnphase 12 aus dieser Gülle. Die abgetrennte Dickphase 11 kann zur Nachvergasung wieder dem Gärsubstrat von Biogasanlagen 10 zugemischt werden.
Die wässrige Dünnphase hingegen ist wertvoller Düngestoff für die extrem schnellwüchsigen aquatischen Kulturpflanzen in der Kultureinrichtung 1.
In der Kultureinrichtung fallen bspw durch den Tageswechseltemperaturzyklus große Mengen an kondensiertem Wasser an. Dies wird in der geschlossenen Kultureinrichtung 1 aufgefangen und rezykliziert in den Kulturwassermischer.
Auf diese Weise wird der wesentliche Teil des in der Kultureinrichtung benötigten Wassers so im Kreis gefahren. Wasserverluste können mit Brunnenwasser ausgeglichen werden, oder aber die Dünnphase bringt genügend Wasser mit.

Zusätzlich oder ersatzweise kann der Kulturwassermischer aber auch von einer Düngeeinrichtung 15 gespeist werden, die als Dünger entweder normaler NPK-Dünger ist (Stickstoff, Phosphor, Kalium) und/oder Reste/Abfälle aus der Lebensmittel-, oder sogar der Futtermittelproduktion, und/oder sogenannte Vinasse. Auf diese Weise gewinnt das Verfahren einen weiteren Punkt in Bezug auf die Nachhaltigkeit der ökologischen Futtermittelproduktion.
Die Viehhaltung brauch natürlich nicht am selben Standort sein. Dennoch entsteht bei dieser zumindest orstnah möglichen Betriebsweise insbesondere in landwirtschaftlichen Genossenschaften ein Rohstoffkreislauf in Gänze, bei dem die für die Futterproduktion aufgewendete Gesamtenergie deutlich reduziert und ebenso nachhaltig mit Wasserressourcen gehandhabt wird.

Im Resultat sinkt auch zusätzlich noch die damit für jedes Kilo Rind-, Schwein-, oder Geflügelfleisch aufgewendete Frischwassermenge dramatisch.

Da diese Kultivierung von aquatischen Pflanzen, inbesondere Wasserlinsen (Lemnaceaen etc) im vorliegenden Verfahren in geschlossenen Kultureinrichtungen erfolgt, werden alle für die Futtermittverwertung gestellten gesetzlichen Anfordnerungen auf einfache Weise erfüllt.

Dieser nachhaltige Umgang mit der Ressource Wasser ist neben den Klimazielen eines der vordringlichen Ziele der nächsten Jahrzehnte.

Außerdem sind die Standorte für solche Einrichtungen wegen des eigentlichen Kulturverfahrens in geschlossenen übereinander gestapelten Kulturwannen nicht nur als freistehende Photobioreaktoren denkbar, sondern auch in Bodeneinsenkungen, oder sogar in alten Stollen, sofern diese zumindest unter den für Wasserlinsen benötigten Schwachlichtbedingungen beleuchtet werden.

### Bezugszeichen:

- 1: Aquatische Pflanzenkultureinrichtung
- 2: Trocknung/Vortrocknung
- 3: Futtermittelproduktionseinrichtung
- 4: Steuereinrichtung
- 5: Steuerungsparameter Beerntung
- 6: Steuerungsparameter Trocknung
- 7: Steuerungsparameter Futtermittelproduktion
- 8: Viehhaltung
- 9: Landwirtschaftliche Gülle
- 10: Biogasanlage
- 11: Dickphasenabtrennung
- 12: Dünnphasenabtrennung
- 13: Kulturwassermischer
- 14: Kondenswasserspeicher
- 15: Dünger/Düngeeinrichtung

## Patentansprüche

1. Verfahren zum Betrieb einer aquatischen Pflanzenkultureinrichtung für die Futtermittel-Produktion, bei welcher in einem geschlossenen Photobioreaktor in einem gestapelten Aquakultursystem aquatische Pflanzen kultiviert und in kurzen wiederholenden Erntezyklen teilbeerntet werden, **dadurch gekennzeichnet, dass** der Pflanzenproduktion im gestapelten Aquakultursystem eine damit zeitlich korrelierte und koordinierte Futtermittelproduktion und/oder Futtermittelkonditionierung beigeordnet ist, derart, dass die Beerntungsmengen der Aquakulturen an die nachfolgende Futtermittelproduktion automatisch in der Weise angepasst ist, dass die in Summe täglich oder wöchentlich beernteten Mengen aquatischer Pflanzen *in situ* zumindest nach teilweiser Auftrocknung der Pflanzen zu Futtermittel verarbeitet werden können, in der Art, dass das besagte aquatische Kultursystem als jahreszeitenunabhängiges ganzjähriges lebendes, bedarfsgeregeltes Frischlager für Biomasse betrieben wird, und dass die Beerntung der Kultureinrichtung und die Futtermittelproduktion über aufeinander abgestimmte Steuerungsparamter gesteuert werden, und dass die aquatische Pflanzenkultureinrichtung in Zonen unterschiedlicher Temperatur und/ oder unterschiedlicher Wärme im Kulturwasser, und/oder unterschiedlicher Ausleuchtung oder Kunstlicht unterschiedlicher Wellenlänge und/oder unterschiedlicher Kulturwasser-zusammensetzung unterteilt ist, in welchen in den aquatischen Pflanzen durch Steuerung dieser Parameter unterschiedliche Inhaltsstoffspektren generierbar sind, und dass zonenweise geerntet wird und die dort jeweils reproduzierbar unterschiedlichen Inhaltsstoffe nachfolgend entsprechend in die Futtermittelproduktion eingehen, um jeweils anforderungsnah designte Biomasse als Rohstoffe zu generieren.

2. Verfahren nach Anspruch 1, **dadurch gekennzeichnet,**
**dass** die bei der Kultivierung benötigten Wassermengen zumindest teilweise aus der Kondensation von Kulturwasserverdunstungsmengen im Photobioreaktor und/oder aus der Auftrocknung der entnommenen Pflanzen wieder in die Kultureinrichtung des Photobioreaktors zurück-geführt wird.

3. Verfahren nach einem der vorhergehenden Ansprüche,
**dadurch gekennzeichnet,**
**dass** die aquatische Kultureinrichtung mittels Abwärme aus dem Futtermittelproduktionsprozess oder mittels Solarthermie beheizt wird.

4. Verfahren nach einem der vorhergehenden Ansprüche,
**dadurch gekennzeichnet,**
**dass** am Frischmasseausschleusungspunkt der aquatischen Kultureinrichtung ein geschlossenes lichtdurchlässig eingehaustes Areal zur solarthermischen Vortrockung der Frischmasse eingesetzt wird.

5. Verfahren nach einem der vorhergehenden Ansprüche,
**dadurch gekennzeichnet,**
**dass** das Kondenswasser aus der aquatischen Kultureinrichtung oder der Vortrocknung in das Kulturwasser der aquatischen Kultureinrichtung rückgeführt führt, indem es kurz vor Zuleitung in die Kulturflächen mit einer Entkeimung behandelt wird.

6. Verfahren nach Anspruch 5, **dadurch gekennzeichnet,**
**dass** landwirtschaftliche Gülle dem Kulturwasser beigemischt wird bevor es entkeimt und nachfolgend der Kultureinrichtung zugeleitet wird.

7. Verfahren nach Anspruch 5 oder 6, **dadurch gekennzeichnet, dass** zuvor aus landwirtschaftlicher Gülle oder Gärsubstrate die Dickphase (feste sedimentierbare Bestandteile) von der Gülle getrennt wird, und die so erhaltene Dünnphase dem Kulturwasser beigemischt wird.

8. Einrichtung mit Pflanzenkultureinrichtung zur Durchführung des Verfahrens nach einem der Ansprüche 1 bis 9, wobei eine geschlossene aquatische Pflanzen-kultureinrichtung mit übereinander gestapelten Kulturwannen eines Aquakultursystems vorgesehen ist, **dadurch gekennzeichnet, dass** der aquatischen Pflanzenkultureinrichtung eine Futtermittelproduktionseinrichtung räumlich beigeordnet oder zugeordnet ist, derart, dass eine damit zeitlich korrelierte und koordinierte Futtermittelproduktion erfolgt, bei welcher die Beerntungsmengen der Aquakulturen an die nachfolgende Futtermittelproduktion automatisch steuerbar ist, und die so in Summe täglich oder wöchentlich beernteten Mengen aquatischer Pflanzen ganzjährig und in situ zumindest nach teilweiser Auftrocknung der Pflanzen direkt zu Futtermittel verarbeitbar ist, und dass die Beerntung der Kultureinrichtung sowie die Futtermittelproduktion über eine gemeinsame Steuerung oder zumindest über aufeinander abgestimmte Steuerungsparameter steuerbar ist, bzw sind, und dass die aquatische Pflanzenkultur-einrichtung in Zonen unterschiedlicher Temperatur und/ oder unterschiedlicher Wärme im Kulturwasser, und/oder unterschiedlicher Ausleuchtung oder Kunstlichtauslicht unterschiedlicher Wellenlänge und/oder unterschiedlicher Kulturwasser-zusammensetzung unterteilt ist, in welchen in den aquatischen Pflanzen durch Steuerung dieser Parameter unterschiedliche Inhaltsstoffspektren generierbar sind, und dass zonenweise geerntet wird und die dort jeweils reproduzierbar unterschiedlichen Inhaltsstoffe nachfolgend entsprechend in die Futtermittelproduktion eingehen, um jeweils anforderungsnah designte Biomasse als Rohstoffe zu generieren.

9. Einrichtung mit Pflanzenkultureinrichtung nach Anspruch 8,
**dadurch gekennzeichnet,**
**dass** am Frischmasseausschleusungspunkt der aquatischen Pflanzenkultureinrichtung ein geschlossenes eingehaustes Areal zur solarthermischen oder prozessabwärmegespeisten Vortrockung der Frischmasse vorgesehen ist.

10. Einrichtung mit Pflanzenkultureinrichtung nach Anspruch 8 oder 9,
**dadurch gekennzeichnet,**
**dass** die Kultureinrichtung sowie das zur Vortrockung vorgesehene Areal mit Kondensationsflächen versehen sind, die flüssigkeits- und/oder tropfschlüssig mit einer Kondensationswassersammelbehältnis verbunden sind, so dass die bei der Kultivierung benötigten Wassermengen zumindest teilweise aus der Kondensation von Kulturwasserverdunstungsmengen im Photobioreaktor und/oder aus der Auftrocknung der entnommenen Pflanzen wieder in die Kultureinrichtung des Photobioreaktors zurückgeführt wird.

11. Einrichtung mit Pflanzenkultureinrichtung nach einem der vorhergehenden Ansprüche 8 bis 10,
**dadurch gekennzeichnet,**
**dass** die aquatische Kultureinrichtung mittels Abwärme aus dem Futtermittelproduktionsprozess oder mittels Solarthermie beheizt wird.

12. Einrichtung mit Pflanzenkultureinrichtung nach einem der vorhergehenden Ansprüche,
**dadurch gekennzeichnet,**
**dass** am Frischmasseausschleusungspunkt der aquatischen Kultureinrichtung ein geschlossenes lichtdurchlässig eingehaustes Areal zur solarthermischen Vortrockung der Frischmasse eingesetzt wird.

13. Einrichtung mit Pflanzenkultureinrichtung nach einem der vorhergehenden Ansprüche,
**dadurch gekennzeichnet,**
**dass** das Kondenswasser aus der aquatischen Kultureinrichtung oder der Vortrocknung in das Kulturwasser der aquatischen Kultureinrichtung rückgeführt führt, indem es kurz vor Zuleitung in die Kulturflächen mit einer Entkeimung behandelt wird.

14. Einrichtung mit Pflanzenkultureinrichtung nach einer der vorhergehenden Ansprüche,
**dadurch gekennzeichnet,**
**dass** landwirtschaftliche Gülle dem Kulturwasser beigemischt wird, bevor es entkeimt und nachfolgend der Kultureinrichtung zugeleitet wird.

15. Einrichtung mit Pflanzenkultureinrichtung nach einer der vorhergehenden Ansprüche,
**dadurch gekennzeichnet,**
**dass** zuvor aus landwirtschaftlicher Gülle die Dickphase (feste sedimentierbare Bestandteile) von der Gülle getrennt wird, und die so erhaltene Dünnphase dem Kulturwasser beigemischt wird.

## Claims

1. Method for operating an aquatic plant culture unit for the production of feedstuff, where aquatic plants are grown, and partially harvested in short repeating harvest cycles, in a closed photobioreactor in a stacked aquaculture system, **characterized in that** the plant production in the stacked aquaculture system has assigned to it a feedstuff production and/or feedstuff conditioning that is time-correlated and coordinated with the plant production, such that the amounts harvested from the aquacultures are automatically adapted to the subsequent feedstuff production, **in that** the sum of the daily or weekly harvested amounts of aquatic plants can be processed *in situ* after at least partial drying of the plants to give feedstuffs, such that said aquatic culture system is operated as a seasonally independent, year-round live demand-driven fresh store for biomass, and **in that** the harvesting of the culture unit and the feedstuff production are controlled via control parameters which are matched to each other, and **in that** the aquatic plant culture unit is divided into zones with a different temperature and/or different warmth in the culture water, and/or different illumination or artificial light with a different wavelength and/or a different culture water composition, in which, by controlling these parameters, different constituent spectra may be generated in the aquatic plants, and **in that** harvesting takes place zone-wise and the constituents, which are in each case reproducibly different there, subsequently enter the feedstuff production accordingly so as to generate, as raw materials, biomass which is in each case designed to approximate the demands.

2. Method according to Claim 1, **characterized in that** the amounts of water required in the cultivation is returned at least in part from the condensation of amounts of evaporated culture water in the photobioreactor and/or from the drying process of the removed plants back to the culture unit of the photobioreactor.

3. Method according to one of the preceding claims, **characterized in that** the aquatic culture unit is heated by waste heat from the feedstuff production process or by means of solar heating.

4. Method according to one of the preceding claims, **characterized in that** a closed transparently housed area for predrying the fresh matter by solar heating is employed at the fresh-matter discharge point of the aquatic culture unit.

5. Method according to one of the preceding claims, **characterized in that** the water condensation from the aquatic culture unit or from the predrying process is recirculated into the culture water of the aquatic culture unit by being treated with a decontamination shortly before being passed into the culture areas.

6. Method according to Claim 5, **characterized in that** agricultural slurry is admixed to the culture water before it is decontaminated and subsequently passed to the culture unit.

7. Method according to Claim 5 or 6, **characterized in that** the thick phase (solid sedimentable components) is separated from the slurry of agricultural slurry or fermentation substrates beforehand, and the resulting thin phase is admixed to the culture water.

8. Unit with a plant culture unit for carrying out the method according to one Claims 1 to 9, wherein a closed aquatic plant culture unit with culture troughs of an aquaculture system, stacked one above the other, is provided, **characterized in that** a feedstuff production unit is spatially associated with or assigned to the aquatic plant culture unit such that a feedstuff production which is time-correlated and coordinated therewith takes place, where the amounts harvested from the aquacultures can be controlled automatically in respect of the subsequent feedstuff production, and the sum of the daily or weekly harvested amounts of aquatic plants, at least after partially drying the plants, can be processed year-round and in situ to give feedstuffs, and **in that** the harvesting of the culture unit and the feedstuff production can be controlled via joint controlling or at least via control parameters which are matched to each other, and **in that** the aquatic plant culture unit is divided into zones with a different temperature and/or different warmth in the culture water, and/or different illumination or artificial light with a different wavelength and/or a different culture water composition, in which, by controlling these parameters, different constituent spectra may be generated in the aquatic plants, and **in that** harvesting takes place zone-wise and the constituents, which are in each case reproducibly different there, subsequently enter the feedstuff production accordingly so as to generate, as raw materials, biomass which is in each case designed to approximate the demands.

9. Unit with a plant culture unit according to Claim 8, **characterized in that** a closed housed area for predrying the fresh matter by solar heating or by waste process heat is provided at the fresh matter discharge point of the aquatic plant culture unit.

10. Unit with a plant culture unit according to Claim 8 or 9, **characterized in that** the culture unit and the area provided for the predrying process are provided with condensation areas which are connected to a container for collecting water condensation such that they are connected via liquid and/or drops, so that the amounts of water required in the cultivation are recirculated at least in part from the condensation of the amounts of evaporated culture water in the photobioreactor and/or from the drying process of the removed plants back into the culture unit of the photobioreactor.

11. Unit with a plant culture unit according to one of the preceding Claims 8 to 10, **characterized in that** the aquatic culture unit is heated by means of waste heat from the feedstuff production process or by means of solar heating.

12. Unit with a plant culture unit according to one of the preceding claims, **characterized in that** a closed transparently housed area for predrying the fresh matter by solar heating is employed at the fresh matter discharge point of the aquatic culture unit.

13. Unit with a plant culture unit according to one of the preceding claims, **characterized in that** the water condensation from the aquatic culture unit or from the predrying process is recirculated into the culture water of the aquatic culture unit by being treated with a decontamination shortly before being passed into the culture areas.

14. Unit with a plant culture unit according to one of the preceding claims, **characterized in that** agricultural slurry is admixed to the culture water before it is decontaminated and subsequently passed to the culture unit.

15. Unit with a plant culture unit according to one of the preceding claims, **characterized in that** the thick phase (solid sedimentable components) is separated from the slurry of agricultural slurry beforehand, and the resulting thin phase is admixed to the culture water.

## Revendications

1. Procédé pour le fonctionnement d'un dispositif de culture aquatique de plantes pour la production d'aliments pour animaux, dans lequel des plantes aquatiques sont cultivées dans un photo-bioréacteur fermé dans un système d'aquaculture empilé et récoltées partiellement en des cycles de récolte récurrents courts, **caractérisé en ce qu'**une production d'aliments pour animaux et/ou un conditionnement d'aliments pour animaux corrélé(e) et coordonné(e) dans le temps est associé(e) à la production de plantes dans le système d'aquaculture empilé de manière telle que les quantités récoltées des aquacultures sont automatiquement adaptées à la production consécutive d'aliments pour animaux de manière telle que les quantités de plantes aquatiques récoltées au total par jour ou par semaine peuvent être transformées en aliments pour animaux in situ au moins après séchage partiel des plantes, de manière telle que ledit système de culture aquatique est exploité en tant qu'entreposage frais pour une biomasse, vivant toute l'année indépendamment de la saison, régulé en fonction des besoins et **en ce que** la récolte du dispositif de culture et la production d'aliments pour animaux sont régulés via des paramètres de régulation adaptés les uns aux autres et **en ce que** le dispositif de culture aquatique de plantes est divisé en zones de température différente et/ou de chaleur différente dans l'eau de culture et/ou d'éclairage différent ou d'éclairage artificiel de différentes longueurs d'onde et/ou de composition différente d'eau de culture, dans lesquelles des spectres de constituants différents peuvent être générés dans les plantes aquatiques par régulation de ces paramètres et **en ce qu'**on récolte par zones et les constituants différents, qui y sont à chaque fois reproductibles, entrent ensuite de manière correspondante dans la production d'aliments pour animaux pour générer une biomasse à chaque fois conçue selon les exigences comme matières premières.

2. Procédé selon la revendication 1, **caractérisé en ce que** les quantités d'eau nécessaires lors de la culture provenant au moins partiellement de la condensation de quantités d'évaporation d'eau de culture dans le photo-bioréacteur et/ou du séchage des plantes prélevées est de nouveau recyclées dans le dispositif de culture du photo-bioréacteur.

3. Procédé selon l'une quelconque des revendications précédentes, **caractérisé en ce que** le dispositif de culture aquatique est chauffé au moyen de chaleur perdue provenant du procédé de production d'aliments pour animaux ou au moyen d'énergie héliothermique.

4. Procédé selon l'une quelconque des revendications précédentes, **caractérisé en ce qu'**une zone à enceinte fermée, transparente à la lumière est utilisée au point de soutirage de masse fraîche du dispositif de culture aquatique pour le préséchage, de manière héliothermique, de la masse fraîche.

5. Procédé selon l'une quelconque des revendications précédentes, **caractérisé en ce que** l'eau de condensation du dispositif de culture aquatique ou du préséchage est recyclée dans l'eau de culture du dispositif de culture aquatique **en ce qu'**elle est traitée par une désinfection peu avant l'alimentation dans les surfaces de culture.

6. Procédé selon la revendication 5, **caractérisé en ce que** du lisier agricole est mélangé à l'eau de culture avant sa désinfection et son introduction consécutive dans le dispositif de culture.

7. Procédé selon la revendication 5 ou 6, **caractérisé en ce que** la phase épaisse (constituants solides pouvant sédimenter) est séparée au préalable du lisier agricole ou du substrat de fermentation et la phase légère ainsi obtenue est mélangée à l'eau de culture.

8. Dispositif présentant un dispositif de culture de plantes pour la réalisation du procédé selon l'une quelconque des revendications 1 à 9, un dispositif fermé de culture aquatique de plantes présentant des cuves de culture empilées les unes sur les autres d'un système de culture aquatique étant prévu, **caractérisé en ce qu'**un dispositif de production d'aliments pour animaux est associé ou affecté dans l'espace au système de culture aquatique de plantes, de manière telle qu'une production corrélée et coordonnée dans le temps est réalisée avec celui-ci, lors de laquelle les quantités récoltées des aquacultures sont automatiquement régulées par rapport à la production consécutive d'aliments pour animaux et les quantités de plantes aquatiques ainsi récoltées au total par jour ou par semaine peuvent être transformées, toute l'année et in situ, directement en aliments pour animaux, au moins après séchage partiel des plantes, et **en ce que** la récolte du dispositif de culture ainsi que la production d'aliments pour animaux peuvent être régulés via une régulation commune ou au moins via des paramètres de régulation adaptés les uns aux autres et **en ce que** le dispositif de culture aquatique de plantes est divisé en zones de température différente et/ou de chaleur différente dans l'eau de culture et/ou d'éclairage différent ou d'éclairage artificiel de différentes longueurs d'onde et/ou de composition différente d'eau de culture, dans lesquelles des spectres de constituants différents peuvent être générés dans les plantes aquatiques par régulation de ces paramètres et **en ce qu'**on récolte par zones et les constituants différents, qui y sont à chaque fois reproductibles, entrent ensuite de manière correspondante dans la production d'aliments pour animaux pour générer une biomasse à chaque fois conçue selon les exigences comme matières premières.

9. Dispositif présentant le dispositif de culture de plantes selon la revendication 8, **caractérisé en ce qu'**une zone à enceinte fermée est prévue au point de soutirage de masse fraîche du dispositif de culture aquatique de plus pour le préséchage, de manière héliothermique ou par injection de chaleur perdue du procédé, de la masse fraîche.

10. Dispositif présentant le dispositif de culture de plantes selon la revendication 8 ou 9, **caractérisé en ce que** le dispositif de culture ainsi que la zone prévue pour le préséchage sont pourvus de surfaces de condensation qui sont reliées en communication fluidique et/ou par des gouttes à un récipient de récupération d'eau de condensation, de manière telle que les quantités d'eau nécessaires lors de la culture provenant au moins partiellement de la condensation de quantités d'évaporation d'eau de culture dans le photo-bioréacteur et/ou du séchage des plantes prélevées sont de nouveau recyclées dans le dispositif de culture du photo-bioréacteur.

11. Dispositif présentant le dispositif de culture de plantes selon l'une quelconque des revendications précédentes 8 à 10, **caractérisé en ce que** le dispositif de culture aquatique est chauffé au moyen de chaleur perdue provenant du procédé de production d'aliments pour animaux ou au moyen d'énergie héliothermique.

12. Dispositif présentant le dispositif de culture de plantes selon l'une quelconque des revendications précédentes, **caractérisé en ce qu'**une zone à enceinte fermée, transparente à la lumière est utilisée au point de soutirage de masse fraîche du dispositif de culture aquatique pour le préséchage, de manière héliothermique, de la masse fraîche.

13. Dispositif présentant le dispositif de culture de plantes selon l'une quelconque des revendications précédentes, **caractérisé en ce que** l'eau de condensation du dispositif de culture aquatique ou du préséchage est recyclée dans l'eau de culture du dispositif de culture aquatique **en ce qu'**elle est traitée par une désinfection peu avant l'alimentation dans les surfaces de culture.

14. Dispositif présentant le dispositif de culture de plantes selon l'une quelconque des revendications précédentes, **caractérisé en ce que** du lisier agricole est mélangé à l'eau de culture avant sa désinfection et son introduction consécutive dans le dispositif de culture.

15. Dispositif présentant le dispositif de culture de plantes selon l'une quelconque des revendications précédentes, **caractérisé en ce que** la phase épaisse (constituants solides pouvant sédimenter) est séparée au préalable du lisier agricole et la phase légère ainsi obtenue est mélangée à l'eau de culture.
